# EUROPÄISCHE PATENTSCHRIFT

(11) **EP 3 057 570 B1**
(45) Veröffentlichungstag und Bekanntmachung des Hinweises auf die Patenterteilung: **18.07.2018**
(21) Anmeldenummer: 14796690.7
(22) Anmeldetag: 26.09.2014
(51) Int. Cl.: A61K 8/73, A61Q 15/00, A61K 8/26, C11D 3/00, C11D 3/22

(54) **VERWENDUNG VON POLYSACCHARIDEN IN SCHWEISSHEMMENDEN KOSMETISCHEN MITTELN ZUR TEXTILSCHONUNG**
USE OF POLYSACCHARIDES IN ANTIPERSPIRANTS FOR PROTECTING TEXTILES
UTILISATION DES POLYSACCHARIDES DANS DES ANTIPERSPIRANTS POUR LA PROTECTION DE TEXTILES

(30) Priorität: 15.10.2013 DE 102013220767
(43) Veröffentlichungstag der Anmeldung: 24.08.2016
(73) Patentinhaber: Henkel AG & Co. KGaA, 40589 Düsseldorf (DE)
(72) Erfinder: BANOWSKI, Bernhard, 40597 Düsseldorf (DE); CLAAS, Marcus, 40723 Hilden (DE); BREUER, Imme, 40474 Düsseldorf (DE)
(86) Internationale Anmeldenummer: PCT/DE2014/200518
(87) Internationale Veröffentlichungsnummer: WO 2015/055201

(56) Entgegenhaltungen:
- EP-A2- 0 676 192
- WO-A2-2013/092185
- DE-A1-102011 083 872

## Beschreibung

Die vorliegende Erfindung betrifft eine Verwendung mindestens eines Polysaccharids in schweißhemmenden kosmetischen Mitteln zur Reduzierung und/oder Vermeidung von Textilverschmutzungen und/oder Textilverfärbungen.

Das Waschen, Reinigen und Pflegen des eigenen Körpers stellt ein menschliches Grundbedürfnis dar und die moderne Industrie versucht fortlaufend, diesen Bedürfnissen des Menschen in vielfältiger Weise gerecht zu werden. Besonders wichtig für die tägliche Hygiene ist die anhaltende Beseitigung oder zumindest Reduzierung des Körpergeruchs und der Achselnässe. Im Stand der Technik sind zahlreiche spezielle deodorierende oder schweißhemmende Körperpflegemittel bekannt, welche für die Anwendung in Körperregionen mit einer hohen Dichte von Schweißdrüsen, insbesondere im Achselbereich, entwickelt wurden. Diese sind in den unterschiedlichsten Darreichungsformen konfektioniert, beispielsweise als Puder, in Stiftform, als Aerosolspray, Pumpspray, flüssige und gelförmige Roll-on-Applikation, Creme, Gel und als getränkte flexible Substrate (Deotücher).

Kosmetische Antitranspirantien des Standes der Technik enthalten neben mindestens einem Öl oder einer Fettsubstanz und einer Riechstoffkomponente bzw. einem Parfüm mindestens ein schweißhemmendes Salz.

Bei regelmäßiger Anwendung können Antitranspirantien zu deutlich sichtbaren farbigen Textilanschmutzungen führen. Oft handelt es sich hierbei um weiße und/oder fettige und/oder gelbe Flecken, welche auch durch intensives Waschen nicht entfernt werden können. Die Fleckenbildung basiert auf einer komplexen Interaktion von Rezepturbestandteilen des Antitranspirants, Schweiß und dem verwendeten Waschmittel. Wahrscheinlich bilden sich zunächst unlösliche Aluminiumverbindungen auf und innerhalb der Faser. Die Gelbfärbung tritt in der Regel mit einer Zeitverzögerung ein und wird zumindest teilweise durch Oxidation ungesättigter Fettsäuren oder anderer organischer Verbindungen hervorgerufen, welche als unlösliche Aluminiumsalze oder auf dem Fleck fixiert vorliegen. Die Intensität der Fleckenbildung auf dem Textil ist hierbei insbesondere abhängig von der Zusammensetzung des Antitranspirant-Produkts, der Art des Parfumöls in dem Antitranspirant, des Waschmittels und der individuellen Schweißmenge und - zusammensetzung.

Weiterhin können bestimmte Bestandteile der Formulierung, beispielsweise hydrophobe Maskierungsmittel, zu dunklen, fettigen/öligen Flecken auf dem Textil führen. Die hydrophoben Maskierungsmittel in Form von kosmetischen Ölen oder Polyolen werden eingesetzt, um weiße Rückstände des Antitranspirants auf dunklen Textilien, welche beispielsweise durch Transfer der Antitranspirantien von der Haut auf ein Textil beim Anziehen entstehen können, zu vermeiden. Jedoch lassen sich diese Maskierungsmittel je nach chemischer Zusammensetzung nur teilweise oder gar nicht durch einen Standardwaschprozess entfernen, so dass die zuvor genannten Flecken entstehen, welche auch die Haptik der Textilien im angeschmutzen Bereich verändern können.

Darüber hinaus können durch die Interaktion von Waschmitteln und Antitranspirantwirkstoffen weitere unlösliche Verbindungen entstehen, welche auf das Textil aufziehen können. Diese unlöslichen Verbindungen bilden weiße, harte Rückstände, welche sich meist erst nach mehreren Anschmutz- und Waschzyklen auf dem Textil zeigen. Diese weißen Rückstände sind in Wasser nicht löslich und auch durch Standard-Waschverfahren nicht zu entfernen. Sie sind besonders gut auf leicht oder dunkel gefärbten Textilien erkennbar.

Um Textilien gegenüber derartigen dauerhaften Verschmutzungen zu schützen, werden im Stand der Technik diverse Inhaltsstoffe zugesetzt. Ein häufig verwendeter Zusatz sind Tenside, wie beispielsweise in dem Dokument WO 2010/097205 A2 beschrieben. Weiterhin ist aus der EP 0 676 192 A2 der Einsatz von hydrophilen Polyurethanen zur Verminderung von Textilflecken bekannt. Auch die Wahl der Ölkomponenten kann die Textilanschmutzung verringern oder aber erhöhen, wie in den Dokumenten US 5 925 338 A, US 4 511 554 A oder US 3 974 270 A beschrieben.

Es besteht daher weiterhin ein Bedarf an Inhaltsstoffen in Antitranspirantien, welche in der Lage sind, die zuvor beschriebenen Verschmutzungen selbst bei der dauerhaften Verwendung der Antitranspirantien effektiv zu reduzieren und/oder zu vermeiden.

Der vorliegenden Erfindung lag die Aufgabe zugrunde, einen Inhaltsstoff in schweißhemmenden kosmetischen Mitteln bereitzustellen, welcher die Nachteile des Standes der Technik vermeidet bzw. zumindest abschwächt und welcher in der Lage ist, Textilverfärbungen und/oder Textilflecken zu reduzieren und/oder zu vermeiden ohne die Wirkung des schweißhemmenden kosmetischen Mittels oder dessen Lagerstabilität negativ zu beeinflussen.

Es wurde nun überraschend gefunden, dass die Verwendung von speziellen Polysacchariden in schweißhemmenden kosmetischen Mitteln zu einer Reduzierung und/oder Vermeidung von Textilverschmutzungen und/oder Textilflecken führt, ohne jedoch die Wirkung des schweißhemmenden kosmetischen Mittels sowie dessen Lagerstabilität negativ zu beeinflussen.

Gegenstand der vorliegenden Erfindung ist somit eine Verwendung eines Polysaccharids, ausgewählt aus der Gruppe von C₁₋₁₀-alkylcellulosen, Hydroxy-C₂₋₁₀-alkylmethylcellulosen, Hydroxy-C₂₋₁₀-alkylethylcellulosen, Hydroxy-C₁₋₁₀-alkylcellulosen, Carboxy-C₁₋₁₀-alkylcelluosen und C₆₋₃₀-Alkylhydroxyethylcellulosen in schweißhemmenden kosmetischen Mitteln zur Reduzierung und/oder Verhinderung von während des Waschvorgangs auftretenden Textilverfärbungen und/oder Textilflecken.

Die Verwendung dieser Polysaccharide in schweißhemmenden kosmetischen Mitteln verhindert - ohne sich auf diese Theorie beschränken zu wollen - eine Wechselwirkung der an dem Textil nach Auftragen des schweißhemmenden kosmetischen Mittels anhaftenden Rückstände des schweißhemmenden Aluminiumsalzes mit bei dem Waschen verwendeten Waschmitteln. Somit wird durch den Zusatz dieser Polysaccharide in schweißhemmenden kosmetischen Mittel die Entstehung von schwerlöslichen Aluminiumverbindungen während des Waschvorgangs verhindert bzw. vermindert und auf diese Weise die Bildung von Textilverfärbungen und/oder Textilflecken signifikant verringert. Weiterhin führt die Zugabe dieser Polysaccharide zu schweißhemmenden kosmetischen Mitteln nicht zu einer negativen Beeinflussung der schweißhemmenden Wirkung dieser Mittel oder zu einer verminderten Lagerungsstabilität dieser Mittel.

Weiterhin wird unter dem Begriff "schweißhemmendes kosmetisches Mittel" im Rahmen der vorliegenden Erfindung ein kosmetisches Mittel verstanden, welches in der Lage ist, die Transpiration der Schweißdrüsen des Körpers zu vermindern bzw. zu reduzieren.

Darüber hinaus wird unter dem Begriff "Textilverfärbung" bzw. unter dem Begriff "Textilfleck" im Rahmen der vorliegenden Erfindung eine Anschmutzung des Textils durch schweißhemmende kosmetische Mittel verstanden, wobei die Anschmutzung des Textils weiß und/oder fettig und/oder gelb sein kann.

Erfindungsgemäß ist das Polysaccharid ausgewählt aus der Gruppe von C₁₋₁₀-alkylcellulosen, Hydroxy-C₂₋₁₀-alkylmethylcellulosen, Hydroxy-C₂₋₁₀-alkylethylcellulosen, Hydroxy-C₁₋₁₀-alkylcellulosen, Carboxy-C₁₋₁₀-alkylcelluosen und C₆₋₃₀-Alkylhydroxyethylcellulosen.

Unter C₆₋₃₀-Alkylhydroxyethylcellulosen sind solche Celluloseverbindungen zu verstehen, bei welchen hydrophobe C₆₋₃₀-Alkylgruppen über Ethylenoxideinheiten an das Cellulosegerüst gebunden sind. Aufgrund der Einführung von C₆₋₃₀-Alkylgruppen an Ethylenoxideinheiten des Grundgerüsts weisen diese Cellulosen einen hydrophoben Charakter auf und werden auch als hydrophob modifizierte Hydroxyethylcellulosen bezeichnet.

Besonders gute Ergebnisse im Hinblick auf die Vermeidung und/oder Verminderung von Textilverfärbungen und/oder Textilflecken werden im Rahmen der vorliegenden Erfindung erhalten, wenn das Polysaccharid ausgewählt ist aus der Gruppe von Methylcellulose, Hydroxyethylcellulose, Hydroxyethylmethylcellulose, Hydroxypropylcellulose, Hydroxypropylmethyl-cellulose, Hydroxypropylethylcellulose, Carboxymethylcellulose und Cetylhydroxyethylcellulose. Insbesondere bei dem Einsatz der zuvor genannten Polysaccharide in schweißhemmenden kosmetischen Mitteln wird eine Fleckenbildung auf Textil durch die Bildung von unlöslichen Niederschlägen aufgrund der Wechselwirkung des auf dem Textil haftenden schweißhemmenden Aluminiumsalzes mit dem Waschmittel verhindert bzw. vermindert. Somit resultiert der Einsatz der zuvor genannten Polysaccharide in einer deutlich verminderten Fleckenbildung auf Textilien während dem Waschvorgang. Weiterhin führen die zuvor genannten Polysaccharide nicht zu einer negativen Beeinträchtigung der schweißhemmenden Wirkung des Alumininumsalzes.

Die zuvor angeführten Polysaccharide sind in schweißhemmenden kosmetischen Mitteln enthalten. Diese schweißhemmenden kosmetischen Mittel enthalten bevorzugt
a) mindestens einen Stoff, ausgewählt aus der Gruppe von kosmetischen Ölen, welche bei 20 °C und 1.013 hPa flüssig sind, Riechstoffen und Wachsen,
b) mindestens ein schweißhemmendes Aluminiumsalz in einer Gesamtmenge von 1 bis 80 Gew.-%, bezogen auf das Gesamtgewicht des schweißhemmenden kosmetischen Mittels.

Durch die Verwendung von speziellen Polysacchariden in aluminiumsalzhaltigen schweißhemmenden kosmetischen Mitteln kann eine Verschmutzung von Textil während des Waschvorgangs aufgrund der Bildung von unlöslichen Aluminiumsalzrückständen mit den Inhaltsstoffen des Waschmittels vermieden bzw. vermindert werden. Weiterhin führt die Verwendung der speziellen Polysaccharide nicht zu einer negativen Beeinträchtigung der schweißhemmenden Wirkung des kosmetischen Mittels.

Unter dem Begriff "kosmetisches Öl" im Sinne der vorliegenden Erfindung wird ein für die kosmetische Verwendung geeignetes Öl verstanden, welches mit Wasser nicht mischbar ist. Weiterhin handelt es sich bei dem kosmetischen Öl weder um Riechstoffe, noch um ätherische Öle.

Zudem werden unter dem Begriff "Riechstoffe" im Sinne der vorliegenden Erfindung Substanzen mit einer Molmasse von 74 bis 300 g/mol verstanden, welche mindestens eine osmophore Gruppe im Molekül enthalten und einen Geruch und/oder Geschmack aufweisen, d. h. sie sind in der Lage, die Rezeptoren der Haarzellen des olfaktorischen Systems zu erregen. Osmophore Gruppen sind kovalent an das Molekülgerüst gebundene Gruppen in Form von Hydroxygruppen, Formylgruppen, Oxogruppen, Alkoxycarbonylgruppen, Nitrilgruppen, Nitrogruppen, Azidgruppen etc. In diesem Zusammenhang fallen unter den Begriff "Riechstoffe" im Sinne der vorliegenden Erfindung auch bei 20 °C und 1.013 hPa flüssige Parfümöle, Parfüme, oder Parfümölbestandteile.

Darüber hinaus werden unter dem Begriff "Wachse" im Rahmen der vorliegenden Erfindung Substanzen verstanden, welche bei 20 °C knetbar oder fest bis brüchig hart sind, eine grobe bis feinkristalline Struktur aufweisen und farblich durchscheinend bis opak, aber nicht glasartig sind. Weiterhin schmelzen diese Substanzen über 25 °C ohne Zersetzung, sind wenig oberhalb des Schmelzpunktes leicht flüssig (wenig viskos), weisen eine stark temperaturabhängige Konsistenz und Löslichkeit auf und sind unter leichtem Druck polierbar.

Der Begriff "flüchtiges kosmetisches Öl" bezeichnet erfindungsgemäß kosmetische Öle, welche bei 20 °C und einem Umgebungsdruck von 1.013 hPa einen Dampfdruck von 2,66 Pa bis 40.000 Pa (0,02 bis 300 mm Hg), vorzugsweise von 10 bis 12.000 Pa (0,1 bis 90 mm Hg), weiter bevorzugt von 13 bis 3.000 Pa (0,1 bis 23 mm Hg), insbesondere von 15 bis 500 Pa (0,1 bis 4 mm Hg), aufweisen.

Darüber hinaus werden unter dem Begriff "nichtflüchtige kosmetische Öle" im Sinne der vorliegenden Erfindung kosmetische Öle verstanden, welche bei 20 °C und einem Umgebungsdruck von 1.013 hPa einen Dampfdruck von weniger als 2,66 Pa (0,02 mm Hg) aufweisen.

Weiterhin sind unter dem Begriff der "Fettsäure", wie er im Rahmen der vorliegenden Erfindung verwendet wird, aliphatische Carbonsäuren zu verstehen, welche unverzweigte oder verzweigte Kohlenstoffreste mit 4 bis 40 Kohlenstoffatomen aufweisen. Die im Rahmen der vorliegenden Erfindung eingesetzten Fettsäuren können sowohl natürlich vorkommende als auch synthetisch hergestellte Fettsäuren sein. Weiterhin können die Fettsäuren einfach oder mehrfach ungesättigt sein.

Schließlich sind unter dem Begriff des "Fettalkohols" im Rahmen der vorliegenden Erfindung aliphatische, einwertige, primäre Alkohole zu verstehen, welche unverzweigte oder verzweigte Kohlenwasserstoffreste mit 4 bis 40 Kohlenstoffatomen aufweisen. Die im Rahmen der Erfindung eingesetzten Fettalkohole können auch ein- oder mehrfach ungesättigt sein.

Die Angabe Gew.-% bezieht sich vorliegend, sofern nicht anderes angegeben ist, auf das Gesamtgewicht der schweißhemmenden kosmetischen Mittel ohne gegebenenfalls vorhandenes Treibmittel.

Im Rahmen der vorliegenden Erfindung kann es vorgesehen sein, dass das bei 20°C und 1.013 hPa flüssige kosmetische Öl ausgewählt ist aus der Gruppe von
(i) flüchtigen cyclischen Siliconölen, insbesondere Cyclotrisiloxan, Cyclotetrasiloxan, Cyclopentasiloxan und Cyclohexasiloxan, und linearen Siliconölen mit 2 bis 10 Siloxaneinheiten, insbesondere Hexamethyldisiloxan, Octamethyltrisiloxan, Decamethyltetrasiloxan;
(ii) flüchtigen Nichtsiliconölen, insbesondere flüssigen Paraffinölen und Isoparaffinölen, wie Isodecan, Isoundecan, Isododecan, Isotridecan, Isotetradecan, Isopentadecan, Isohexadecan und Isoeicosan;
(iii) nichtflüchtigen Siliconölen, insbesondere höhermolekularen linearen Polyalkylsiloxane;
(iv) nichtflüchtigen Nichtsiliconölen, insbesondere den Estern von linearen oder verzweigten gesättigten oder ungesättigten C₂₋₃₀-Fettalkoholen mit linearen oder verzweigten gesättigten oder ungesättigten C₂₋₃₀-Fettsäuren, welche hydroxyliert sein können, den C₈-C₂₂-Fettalkoholestern einwertiger oder mehrwertiger C₂-C₇-Hydroxycarbonsäuren, den Triethylcitraten, den verzweigten gesättigten oder ungesättigten C₆₋₃₀-Fettalkoholen, den Mono-, Di- und Triglyceriden von linearen oder verzweigten, gesättigten oder ungesättigten, gegebenenfalls hydroxylierten C₈₋₃₀-Fettsäuren, den Dicarbonsäureestern von linearen oder verzweigten C₂-C₁₀-Alkanolen, den Anlagerungsprodukten von Ethylenoxid und/oder Propylenoxid an ein- oder mehrwertige C_{3.22}-Alkanole, welche gegebenenfalls verestert sein können, den symmetrischen, unsymmetrischen oder cyclischen Estern der Kohlensäure mit Fettalkoholen, den Estern von Dimeren ungesättigter C₁₂₋₂₂-Fettsäuren mit einwertigen, linearen, verzweigten und cyclischen C₂₋₁₈-Alkanolen oder C₂₋₆-Alkanolen, den Benzoesäureestern von linearen oder verzweigten C₈₋₂₂-Alkanolen, wie Benzoesäure-C₁₂₋₁₅-Alkylester und Benzoesäureisostearylester und Benzoesäureoctyldodecylester, den synthetischen Kohlenwasserstoffen, wie Polyisobuten und Polydecene, den alicyclischen Kohlenwasserstoffen; sowie
(v) deren Mischungen.

Der Einsatz von flüchtigen Siliconölen und flüchtigen Nichtsiliconölen im Rahmen der erfindungsgemäßen Verwendung resultiert in einem trockeneren Hautgefühl und in einer schnelleren Freisetzung des schweißhemmenden Aluminiumsalzes.

Die im Rahmen der Erfindung einsetzbaren cyclischen flüchtigen Siliconöle weisen bei 20 °C und einem Umgebungsdruck von 1.013 hPa einen Dampfdruck von 13 bis 15 Pa (0,1 mm Hg) auf. Weiterhin kann erfindungsgemäß als lineares flüchtiges Siliconöl auch ein niedermolekulares Phenyl Trimethicone mit einem Dampfdruck von etwa 2.000 Pa (15 mm Hg) bei 20 °C und einem Umgebungsdruck von 1.013 hPa eingesetzt werden. Aufgrund der hohen Persistenz von Cyclodimethiconen in der Umwelt kann es erfindungsgemäß jedoch bevorzugt sein, wenn in den schweißhemmenden kosmetischen Mitteln 0 bis weniger als 1 Gew.-%, vorzugsweise 0 bis weniger als 0,1 Gew.-%, cyclische flüchtige Siliconöle enthalten sind.

Erfindungsgemäß werden bevorzugt flüchtige Nichtsiliconöle in Form von C₁₀₋₁₃-Isoparaffin-Mischungen mit einem Dampfdruck von 10 bis 400 Pa (0,08 bis 3 mm Hg), vorzugsweise von 13 bis 100 Pa (0,1 bis 0,8 mm Hg), bei 20 °C und einem Umgebungsdruck von 1.013 hPa eingesetzt. Dabei ist es im Rahmen der vorliegenden Erfindung bevorzugt, wenn das flüchtige C₈-C₁₆-Isoparaffin in einer Gesamtmenge von 1 bis 90 Gew.-%, vorzugsweise von 3 bis 45 Gew.-%, bevorzugt von 5 bis 40 Gew.-%, insbesondere von 8 bis 20 Gew.-%, bezogen auf das Gesamtgewicht des schweißhemmenden kosmetischen Mittels, enthalten ist. Selbstverständlich ist es ebenfalls möglich, schweißhemmende kosmetische Mittel mit einem geringen Anteil an flüchtigen Ölen - das heißt, mit 0,1 bis 15 Gew.-%, bezogen auf das Gesamtgewicht des schweißhemmenden kosmetischen Mittels, an flüchtigen Ölen - oder sogar ohne flüchtige Öle zu formulieren.

Zur Maskierung von unlöslichen Bestandteilen, wie Talkum oder auf der Haut angetrockneten schweißhemmenden Aluminiumsalzen, kann es bevorzugt sein, wenn die schweißhemmenden kosmetischen Mittel ein nichtflüchtiges Siliconöl und/oder ein nichtflüchtiges Nichtsiliconöl enthalten.

In diesem Zusammenhang enthalten bevorzugte schweißhemmende kosmetische Mittel mindestens einen Ester der linearen oder verzweigten gesättigten oder ungesättigten Fettalkohole mit 2 bis 30 Kohlenstoffatomen mit linearen oder verzweigten gesättigten oder ungesättigten Fettsäuren mit 2 bis 30 Kohlenstoffatomen, welche hydroxyliert sein können, in einer Gesamtmenge von 1 bis 30 Gew.-%, vorzugsweise von 5 bis 26 Gew.-%, bevorzugt von 9 bis 24 Gew.-%, insbesondere von 12 bis 17 Gew.-%, bezogen auf das Gesamtgewicht des schweißhemmenden kosmetischen Mittels. Es kann jedoch auch vorgesehen sein, dass die schweißhemmenden kosmetischen Mittel mindestens einen Ester der linearen oder verzweigten gesättigten oder ungesättigten Fettalkohole mit 2 bis 30 Kohlenstoffatomen mit linearen oder verzweigten gesättigten oder ungesättigten Fettsäuren mit 2 bis 30 Kohlenstoffatomen, welche hydroxyliert sein können, in einer Gesamtmenge von 1 bis 90 Gew.-%, vorzugsweise von 5 bis 60 Gew.-%, bevorzugt von 9 bis 35 Gew.-%, insbesondere von 12 bis 17 Gew.-%, bezogen auf das Gesamtgewicht des schweißhemmenden kosmetischen Mittels, enthalten.

Im Rahmen der vorliegenden Erfindung können als nichtflüchtige Siliconöle lineare Polyalkylsiloxane mit einer kinematischen Viskosität bei 25 °C von 5 bis 2.000 cSt, insbesondere linearen Polydimethylsiloxane mit einer kinematischen Viskosität bei 25 °C von 5 bis 2.000 cSt, vorzugsweise von 10 bis 350 cSt, insbesondere von 50 bis 100 cSt, eingesetzt werden. Die vorstehend genannten nichtflüchtigen Siliconöle sind unter den Handelsnamen Dow Corning® 200 bzw. Xiameter PMX von Dow Corning bzw. Xiameter erhältlich. Weitere bevorzugte nichtflüchtige Siliconöle sind Phenyltrimethicone mit einer kinematischen Viskosität bei 25 °C von 10 bis 100 cSt, vorzugsweise von 15 bis 30 cSt sowie Cetyldimethicone.

Erfindungsgemäß bevorzugt ist weiterhin der Einsatz von Mischungen der vorstehend genannten kosmetischen Öle, insbesondere von nichtflüchtigen und flüchtigen kosmetischen Ölen, da auf diese Weise Parameter wie Hautgefühl und Stabilität des schweißhemmenden kosmetischen Mittels eingestellt und die Verwendung des Mittels somit besser an die Bedürfnisse der Verbraucher angepasst werden kann.

Im Rahmen der vorliegenden Erfindung ist es bevorzugt, wenn das bei 20 °C und 1.013 hPa flüssige kosmetische Öl in einer Gesamtmenge von 1 bis 95 Gew.-%, vorzugsweise von 10 bis 85 Gew.-%, bevorzugt von 20 bis 75 Gew.-%, weiter bevorzugt von 35 bis 70 Gew.-%, insbesondere von 50 bis 60 Gew.-%, bezogen auf das Gesamtgewicht des schweißhemmenden kosmetischen Mittels, enthalten ist.

Es kann jedoch auch bevorzugt sein, wenn das bei 20 °C und 1.013 hPa flüssige kosmetische Öl in einer Gesamtmenge von 0,2 bis 70 Gew.-%, vorzugsweise von 2 bis 60 Gew.-%, bevorzugt von 3 bis 50 Gew.-%, weiter bevorzugt von 5 bis 35 Gew.-%, insbesondere von 8 bis 20 Gew.-%, bezogen auf das Gesamtgewicht des schweißhemmenden kosmetischen Mittels, enthalten ist. In diesem Zusammenhang kann es auch vorgesehen sein, dass die schweißhemmenden Mittel weniger als 0,2 Gew.-%, vorzugsweise weniger als 0,1 Gew.-%, insbesondere 0 Gew.-% des bei 20 °C und 1.013 hPa flüssigen kosmetischen Öls enthalten. Der Einsatz nur äußerst geringer Mengen des bei 20 °C und 1.013 hPa flüssigen kosmetischen Öls ist insbesondere bei schweißhemmenden kosmetischen Mitteln bevorzugt, welche in wässriger, wässrig-alkoholischer oder wässrig-glykolischer Form vorliegen.

Gemäß einer bevorzugten Ausführungsform der vorliegenden Erfindung ist der Riechstoff ausgewählt aus der Gruppe von
(i) Estern, insbesondere Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenyl-glycinat, Allylcyclohexylpropionat, Styrallylpropionat, Benzylsalicylat, Cyclohexylsalicylat, Floramat, Melusat und Jasmecyclat;
(ii) Ethern, insbesondere Benzylethylether und Ambroxan;
(iii) Aldehyden, insbesondere linearen Alkanale mit 8 bis 18 Kohlenstoffatomen, Citral, Citronellal, Citronellyloxy-acetaldehyd, Cyclamenaldehyd, Lilial und Bourgeonal;
(iv) Ketonen, insbesondere Jonone, alpha-Isomethylionon und Methylcedrylketon;
(v) Alkoholen, insbesondere Anethol, Citronellol, Eugenol, Geraniol, Linalool, Phenylethylalkohol und Terpineol;
(vi) Kohlenwasserstoffen, insbesondere Terpene wie Limonen und Pinen; sowie
(vii) deren Mischungen.
Bevorzugt werden jedoch Mischungen verschiedener Riechstoffe verwendet, welche gemeinsam eine ansprechende Duftnote erzeugen.

Besonders ansprechend riechende schweißhemmende kosmetische Mittel werden erhalten, wenn der Riechstoff in einer Gesamtmenge von 0,00001 bis 10 Gew.-%, vorzugsweise von 0,001 bis 9 Gew.-%, bevorzugt von 0,01 bis 8 Gew.-%, weiter bevorzugt von 0,5 bis 7 Gew.-%, insbesondere von 1 bis 6 Gew.-%, bezogen auf das Gesamtgewicht des schweißhemmenden kosmetischen Mittels, enthalten ist. In diesem Zusammenhang kann es jedoch auch vorgesehen sein, dass der Riechstoff in einer Gesamtmenge von 0,00001 bis 5 Gew.-%, vorzugsweise 0,001 bis 4 Gew.-%, bevorzugt 0,01 bis 3 Gew.-%, weiter bevorzugt 0,1 bis 2 Gew.-%, insbesondere 0,2 bis 1,5 Gew.-%, bezogen auf das Gesamtgewicht des treibmittelhaltigen schweißhemmenden kosmetischen Mittels, enthalten ist.

Gemäß einer weiteren bevorzugten Ausführungsform der vorliegenden Erfindung ist das Wachs ausgewählt aus der Gruppe von
(i) Fettsäureglycerinmono-, -di- und -triestern;
(ii) Butyrospermum Parkii (Shea Butter);
(iii) Estern von gesättigten, einwertigen C₈₋₁₈-Alkoholen mit gesättigten C₁₂₋₁₈-Monocarbonsäuren;
(iv) linearen, primären C₁₂-C₂₄-Alkanolen;
(v) Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat;
(vi) Glycerintriestern von gesättigten linearen C₁₂- C₃₀-Carbonsäuren, die hydroxyliert sein können, insbesondere hydriertes Palmöl, hydriertes Kokosöl, hydriertes Rizinusöl, Glyceryltribehenat und Glyceryltri-12-hydroxystearat;
(vii) natürlichen pflanzlichen Wachsen, insbesondere Candelillawachs, Carnaubawachs, Japanwachs, Zuckerrohrwachs, Ouricourywachs, Korkwachs, Sonnenblumenwachs, Fruchtwachse;
(viii) tierischen Wachsen, insbesondere Bienenwachs, Schellackwachs und Walrat;
(ix) synthetischen Wachsen, insbesondere Montanesterwachse, hydrierte Jojobawachse und Sasolwachse, Polyalkylenwachse und Polyethylenglycolwachse, C₂₀-C₄₀-Dialkylester von Dimersäuren, C₃₀-₅₀-Alkylbienenwachs sowie Alkyl- und Alkylarylester von Dimerfettsäuren, Paraffinwachse; sowie
(x) deren Mischungen.

Besonders bevorzugt sind Handelsprodukte mit der INCI-Bezeichnung Cocoglycerides, insbesondere die Handelsprodukte Novata® (ex BASF), besonders bevorzugt Novata® AB, ein Gemisch aus C₁₂₋₁₈-Mono-, Di- und Triglyceriden, das im Bereich von 30 bis 32°C schmilzt, sowie die Produkte der Softisan-Reihe (Sasol Germany GmbH) mit der INCI-Bezeichnung Hydrogenated Cocoglycerides, insbesondere Softisan 100, 133, 134, 138, 142. Weitere bevorzugte Ester von gesättigten, einwertigen C₁₂₋₁8-Alkoholen mit gesättigten C₁₂₋₁₈-Monocarbonsäuren sind Stearyllaurat, Cetearylstearat (z. B. Crodamol® CSS), Cetylpalmitat (z. B. Cutina® CP) und Myristylmyristat (z. B. Cetiol® MM). Weiterhin wird bevorzugt ein C₂₀-C₄₀-Alkylstearat als Wachskomponente eingesetzt. Dieser Ester ist unter den Namen Kesterwachs® K82H oder Kesterwachs® K80H bekannt und wird von Koster Keunen Inc. vertrieben.

Im Rahmen der vorliegenden Erfindung ist es bevorzugt, wenn das Wachs in einer Gesamtmenge von 0,01 bis 20 Gew.-%, vorzugsweise von 3 bis 20 Gew.-%, bevorzugt von 5 bis 18 Gew.-%, insbesondere von 6 bis 15 Gew.-%, bezogen auf das Gesamtgewicht des schweißhemmenden kosmetischen Mittels, enthalten ist.

Eine besonders gute schweißhemmende Wirkung wird erhalten, wenn das schweißhemmende Aluminiumsalz in einer Gesamtmenge von 2 bis 40 Gew.-%, vorzugsweise von 3 bis 35 Gew.-%, bevorzugt von 4 bis 32 Gew.-%, weiter bevorzugt von 5 bis 28 Gew.-%, noch mehr bevorzugt von 8 bis 25 Gew.-%, insbesondere von 12 bis 22 Gew.-%, bezogen auf das Gesamtgewicht des schweißhemmenden kosmetischen Mittels, enthalten ist. Es kann jedoch auch vorgesehen sein, dass das schweißhemmende Aluminiumsalz in einer Gesamtmenge von 0,1 bis 20 Gew.-%, vorzugsweise von 0,5 bis 15 Gew.-%, bevorzugt von 1 bis 12 Gew.-%, weiter bevorzugt von 1,5 bis 10 Gew.-%, insbesondere von 2 bis 8 Gew.-%, bezogen auf das Gesamtgewicht der treibmittelhaltigen schweißhemmenden Zusammensetzung, enthalten ist.

Im Rahmen der vorliegenden Erfindung kann das schweißhemmende Aluminiumsalz ausgewählt sein aus der Gruppe von
(i) wasserlöslichen adstringierenden anorganischen Salzen des Aluminiums, insbesondere Aluminiumchlorhydrat, Aluminiumsesquichlorohydrat, Aluminiumdichlorohydrat, Aluminium-hydroxid, Kaliumaluminiumsulfat, Aluminiumbromhydrat, Aluminiumchlorid, Aluminiumsulfat;
(ii) wasserlöslichen adstringierenden organischen Salzen des Aluminiums, insbesondere Aluminiumchlorhydrex-Propylenglycol, Aluminiumchlorhydrex-Polyethylenglycol, Aluminium-Propylenglycol-Komplexe, Aluminiumsesquichlorhydrex-Propylenglycol, Aluminiumsesqui-chlorhydrex-Polyethylenglycol, Aluminium-Propylenglycol-dichlorhydrex, Aluminium-Poly-ethylenglycoldichlorhydrex, Aluminiumundecylenoylcollagenaminosäure, Natriumaluminium-lactat, Natriumaluminiumchlorhydroxylactat, Aluminium-lipoaminosäuren, Aluminiumlactat, Aluminiumchlorohydroxyallantoinat, Natrium-Aluminium-Chlorohydroxylactat;
(iii) wasserlöslichen adstringierenden anorganischen Aluminium-Zirkonium-Salzen, insbesondere Aluminiumzirconiumtrichlorhydrat, Aluminiumzirconiumtetrachlorhydrat, Aluminiumzirconiumpentachlorhydrat, Aluminiumzirconiumoctachlorhydrat;
(iv) wasserlöslichen adstringierenden organischen Aluminium-Zirkonium-Salzen, insbesondere Aluminiumzirkonium-Propylenglycol-Komplexe, Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin; sowie
(v) deren Mischungen.

Unter dem Begriff "schweißhemmenden Aluminiumsalze" werden erfindungsgemäß keine Alumosilicate und Zeolithe verstanden. Weiterhin werden erfindungsgemäß unter wasserlöslichen Aluminiumsalzen solche Salze verstanden, welche eine Löslichkeit von mindestens 3 Gew.-% bei 20 °C aufweisen, d. h. es lösen sich mindestens 3 g des schweißhemmenden Aluminiumsalzes in 97 g Wasser bei 20 °C.

Besonders bevorzugte anorganische Aluminiumsalze sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nichtaktivierter (polymerisierter) oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nichtaktivierter (polymerisierter) oder in aktivierter (depolymerisierter) Form vorliegen kann. Die Herstellung derartiger schweißhemmender Aluminiumsalze ist beispielsweise in den Druckschriften US 3 887 692 A, US 3 904 741 A, US 4 359 456 A, GB 2 048 229 A und GB 1 347 950 A offenbart.

Erfindungsgemäß besonders bevorzugte schweißhemmende Aluminiumsalze sind ausgewählt aus sogenannten "aktivierten" Aluminiumsalzen, welche auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in den Druckschriften GB 2 048 229 A, US 4 775 528 A und US 6 010 688 A offenbart. Aktivierte Aluminiumsalze werden in der Regel durch Wärmebehandlung einer verdünnten Lösung des entsprechenden Salzes erzeugt (z.B. einer Lösung mit 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet, werden. Neben der Sprühtrocknung ist z. B. auch die Walzentrocknung geeignet. Aktivierte Aluminiumsalze haben typischerweise ein HPLC-Peak 4-zu-Peak 3-Flächenverhältnis von mindestens 0,4, vorzugsweise von mindestens 0,7, insbesondere von mindestens 0,9, wobei mindestens 70% des Aluminiums diesen HPLC-Peaks zuzuordnen sind.

In diesem Zusammenhang sind ebenfalls "aktiviertes" Aluminium-Zirkoniumsalze bekannt, welche einen hohen HPLC-Peak 5-Aluminium-Gehalt, insbesondere eine Peak 5-Fläche von mindestens 33 %, vorzugsweise von mindestens 45 %, bezogen auf die gesamte Fläche unter den Peaks 2 bis 5, gemessen mit HPLC einer 10 Gew.-%igen wässrigen Lösung des Wirkstoffs unter Bedingungen, bei welchen die Aluminiumspecies in mindestens 4 aufeinander folgende Peaks aufgelöst werden (mit Peaks 2 bis 5 bezeichnet) aufweisen. Bevorzugte Aluminium-Zirconiumsalze mit einem hohen HPLC-Peak 5-Aluminium-Gehalt (auch als "E⁵AZCH" bezeichnet) sind beispielsweise in den Druckschriften US 6 436 381 A und US 6 649 152 A offenbart. Weitern können die vorstehend genannten aktivierten Aluminium-Zirkoniumsalz zusätzlich mit einem wasserlöslichen Strontiumsalz und/oder mit einem wasserlöslichen Calciumsalz stabilisiert sein, wie sie beispielsweise in der Druckschrift US 6 923 952 A offenbart sind.

Es ist erfindungsgemäß ebenfalls möglich, schweißhemmende Aluminiumsalze als nichtwässrige Lösungen oder Solubilisate eines aktivierten schweißhemmenden Aluminium- oder Aluminium-Zirkoniumsalzes, beispielsweise gemäß der Druckschrift US 6 010 688 A, einzusetzen. Derartige Aluminium- bzw. Aluminium-Zirkoniumsalze werden durch den Zusatz einer wirksamen Menge eines mehrwertigen Alkohols, welcher 3 bis 6 Kohlenstoffatome und 3 bis 6 Hydroxylgruppen, bevorzugt Propylenglycol, Sorbit und Pentaerythrit, aufweist, gegen den Verlust der Aktivierung des Salzes stabilisiert.

Besonders bevorzugt sind auch Komplexe aktivierter schweißhemmender Aluminium- bzw. Aluminium-Zirkoniumsalze mit einem mehrwertigen Alkohol, welche 20 bis 50 Gew.-%, vorzugsweise 20 bis 42 Gew.-%, aktiviertes schweißhemmendes Aluminium- bzw. Aluminium-Zirkoniumsalz und 2 bis 16 Gew.-% molekular gebundenes Wasser enthalten, wobei der Rest zu 100 Gew.-% mindestens ein mehrwertiger Alkohol mit 3 bis 6 Kohlenstoffatomen und 3 bis 6 Hydroxylgruppen ist. Propylenglycol, Propylenglycol/Sorbit-Mischungen und Propylenglycol/Pentaerythrit-Mischungen sind bevorzugte derartige Alkohole. Derartige erfindungsgemäß bevorzugte Komplexe eines aktivierten schweißhemmenden Aluminium- bzw. Aluminium-Zirkoniumsalzes mit einem mehrwertigen Alkohol sind z. B. in den Druckschriften US 5 643 558 A und US 6 245 325 A offenbart.

Im Rahmen der vorliegenden Erfindung ist es ebenfalls möglich, als schweißhemmende Aluminiumsalze basische Calcium-Aluminiumsalze, wie sie z. B. in der Druckschrift US 2 571 030 A offenbart sind, einzusetzen. Diese Salze können durch Umsetzung von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid erhalten werden. Es ist jedoch ebenfalls möglich, Aluminium-Zirconium-Komplexe, welche mit Salzen von Aminosäuren, insbesondere mit Alkali- und Erdalkaliglycinaten, gepuffert sind und wie sie z. B. in der Druckschrift US 4 017 599 A offenbart sind, einzusetzen.

Als erfindungsgemäß bevorzugte schweißhemmende aktivierte Aluminium- und Aluminium-Zirkoniumsalze können auch die in den nachfolgenden Druckschriften US 6 245 325 A, US 6 042 816 A, US 6 245 325 A, US 6 042 816 A, US 6 245 325 A, US 6 042 816 A, US 6 245 325 A, US 6 042 816 A oder US 7 105 691 A angeführten Aluminium bzw. Aluminium-Zirkoniumsalze eingesetzt werden, welche bevorzugt durch Aminosäuren, insbesondere Glycin, Hydroxyalkansäuren, insbesondere Glycolsäure und Milchsäure, oder Betaine stabilisiert sind.

Weitere bevorzugte aktivierte Aluminiumsalze sind solche der allgemeinen Formel Al₂(OH)₆₋ₐXa, worin X für Cl, Br, I oder NO₃ und "a" für eine Zahl von 0,3 bis 5, vorzugsweise von 0,8 bis 2,5 insbesondere von 1 bis 2 steht, so dass das Molverhältnis von Al:X 0,9:1 bis 2,1:1 beträgt. Derartige aktivierte schweißhemmende Aluminiumsalze sind beispielsweise in der Druckschrift US 6 074 632 A offenbart. Besonders bevorzugt ist Aluminiumchlorhydrat (d.h. X steht in der vorgenannten Formel für Cl) und speziell 5/6-basisches Aluminiumchlorhydrat mit "a" = 1, so dass das Molverhältnis von Aluminium zu Chlor 1,9 : 1 bis 2,1 : 1 beträgt.

Bevorzugte aktivierte Aluminium-Zirconiumsalze sind solche der allgemeinen Formel ZrO(OH)_{2-pb}Y_{b}, worin Y für Cl, Br, I, NO₃ oder SO₄, b für eine rationale Zahl von 0,8 bis 2 und p für die Wertigkeit von Y steht, so dass das AI:Zr-Molverhältnis von 2 bis 10 und das Metall:(X+Y)-Verhältnis von 0,73 bis 2,1, vorzugsweise von 0,9 bis 1,5 beträgt. Derartige aktivierte schweißhemmende Aluminium-Zirkoniumsalze sind beispielsweise in der zuvor genannten Druckschrift US 6 074 632 A offenbart. Ein besonders bevorzugtes Salz ist Aluminium-Zirconiumchlorhydrat (d.h. X und Y stehen für Cl), welches ein AI:Zr-Verhältnis von 2 bis 10 und ein molares Metall:Cl-Verhältnis von 0,9 bis 2,1 aufweist. Bevorzugte schweißhemmende Wirkstoffe sind in den Druckschriften US 6 663 854 A und US 2004/0009133 A1 offenbart.

Erfindungsgemäß besonders bevorzugte schweißhemmende Aluminiumsalze weisen ein molares Metall-zu-Chlorid-Verhältnis von 1,9 bis 2,1 auf. Das Metall-zu-Chlorid-Verhältnis von im Rahmen der Erfindung ebenfalls besonders bevorzugten Aluminiumsesquichlorohydraten beträgt 1,5 : 1 bis 1,8 : 1. Bevorzugte Aluminium-Zirconiumtetrachlorohydrate weisen ein molares Verhältnis von Al:Zr von 2 bis 6 und von Metall : Chlorid von 0.9 bis 1.3 auf, wobei insbesondere Salze mit einem molaren Metall-zu-Chlorid-Verhältnis von 0,9 bis 1,1, vorzugsweise von 0,9 bis 1,0 bevorzugt sind.

Im Rahmen der vorliegenden Erfindung wird eine besonders gute Reduzierung von Textilverfärbungen und/oder Textilflecken bei der Verwendung des schweißhemmenden kosmetischen Mittels erreicht, wenn das Polysaccharid in einer Gesamtmenge von 0,05 bis 8 Gew.-%, vorzugsweise von 0,1 bis 7 Gew.-%, bevorzugt von 0,3 bis 5 Gew.-%, weiter bevorzugt von 0,5 bis 3 Gew.-%, noch weiter bevorzugt von 0,8 bis 2,5 Gew.-%, insbesondere von 1 bis 2 Gew.-%, bezogen auf das Gesamtgewicht des schweißhemmenden kosmetischen Mittels, enthalten ist. Es ist im Rahmen der vorliegenden Erfindung jedoch auch möglich, dass das Polysaccharid in einer Gesamtmenge von 0,05 bis 20 Gew.-%, vorzugsweise 0,3 bis 18 Gew.-%, bevorzugt 0,5 bis 15 Gew.-%, weiter bevorzugt von 0,7 bis 10 Gew.-%, noch weiter bevorzugt von 1,0 bis 9 Gew.-%, insbesondere von 1,5 bis 6 Gew.-%, bezogen auf das Gesamtgewicht des schweißhemmenden kosmetischen Mittels, enthalten ist. Es kann jedoch auch vorgesehen sein, dass das Polysaccharid in einer Gesamtmenge von 0,05 bis 20 Gew.-%, vorzugsweise 0,3 bis 18 Gew.-%, bevorzugt 0,5 bis 15 Gew.-%, weiter bevorzugt von 0,7 bis 10 Gew.-%, noch weiter bevorzugt von 1,0 bis 9 Gew.-%, insbesondere von 1,5 bis 6 Gew.-%, bezogen auf das Gesamtgewicht des treibmittelhaltigen schweißhemmenden kosmetischen Mittels, enthalten ist. Ohne sich auf diese Theorie beschränken zu wollen resultiert der Einsatz der vorstehend genannten Mengen des Polysaccharids in einer besonders guten Verminderung von Textilflecken und/oder Textilverfärbungen bei der erfindungsgemäßen Verwendung einer Polysaccharid haltigen schweißhemmenden Zusammensetzung, da die zuvor genannten Mengen an Polysacchariden die Bildung von unlöslichen Rückständen auf Textilien aufgrund der Reaktion des Aluminiumsalzes mit Bestandteilen des Waschmittels effektiv vermeiden bzw. vermindern. Weiterhin führt der Einsatz der zuvor genannten Mengen an Polysacchariden nicht zu einer negativen Beeinflussung der schweißhemmenden Wirkung des kosmetischen Mittels.

Im Rahmen einer besonders bevorzugten Ausführungsform beträgt das Gewichtsverhältnis des schweißhemmenden Aluminiumsalzes zu dem Polysaccharid 7 : 1. Darüber hinaus führt auch der Einsatz eines Gewichtsverhältnisses des schweißhemmenden Aluminiumsalzes zu dem Polysaccharid von 10 : 1 zu einer signifikanten Verminderung der Fleckenbildung auf Textilien. Darüber hinaus tritt bei Einsatz der zuvor genannten Gewichtsverhältnisse keine negative Beeinflussung der schweißhemmenden Wirkung sowie der Lagerstabilität des schweißhemmenden kosmetischen Mittels durch den Einsatz von Polysacchariden auf.

Gemäß einer weiteren Ausführungsform der vorliegenden Erfindung enthält das schweißhemmende kosmetische Mittel keine zirkoniumhaltigen Verbindungen.

Das schweißhemmende kosmetische Mittel enthält bevorzugt freies Wasser in einer Gesamtmenge von weniger als 10 Gew.-%, vorzugweise von weniger als 8 Gew.-%, bevorzugt von weniger als 5 Gew.-%, weiter bevorzugt von weniger als 3 Gew.-%, noch mehr bevorzugt von weniger als 1 Gew.-%, insbesondere von 0 Gew.-%, bezogen auf das Gesamtgewicht des schweißhemmenden kosmetischen Mittels. Unter freiem Wasser im Sinne der vorliegenden Erfindung wird somit Wasser verstanden, welches von Kristallwasser, Hydratationswasser oder ähnlich molekular gebundenem Wasser der eingesetzten Bestandteile, insbesondere der schweißhemmenden Aluminiumsalze, verschieden ist.

Überraschenderweise wurde festgestellt, dass signifikant weniger Textilverfärbungen und/oder Textilflecken auftreten, wenn die schweißhemmenden kosmetischen Mittel freies Wasser in einer Menge von 15 bis 96 Gew.-%, bezogen auf das Gesamtgewicht des schweißhemmenden kosmetischen Mittels, enthalten. In einer besonders bevorzugten Ausführungsform der vorliegenden Erfindung enthält das schweißhemmende kosmetische Mittel daher freies Wasser in einer Gesamtmenge von 15 bis 96 Gew.-%, vorzugsweise von 25 bis 80 Gew.-%, bevorzugt von 30 bis 70 Gew.-%, insbesondere von 40 bis 60 Gew.-%, bezogen auf das Gesamtgewicht des schweißhemmenden kosmetischen Mittels.

Die schweißhemmenden kosmetischen Mittel liegen bevorzugt als Suspension des ungelösten schweißhemmenden Aluminiumsalzes in dem bei 20 °C und 1.013 hPa flüssigen kosmetischen Öl vor.

In einer weiteren bevorzugten Darreichungsform liegt das schweißhemmende kosmetische Mittel als Wasser-in-Öl-Emulsion vor. Hierbei kann es sich insbesondere um eine versprühbare Wasser-in-Öl-Emulsion handeln, welche mittels eines Treibmittels versprüht werden kann. In diesem Zusammenhang ist es bevorzugt, wenn in dem in Form einer Wasser-in-ÖI-Emulsion vorliegenden schweißhemmenden kosmetischen Mittel das Polysaccharid in einer Gesamtmenge von 0,05 bis 8 Gew.-%, vorzugsweise von 0,1 bis 7 Gew.-%, bevorzugt von 0,3 bis 5 Gew.-%, weiter bevorzugt von 0,5 bis 3 Gew.-%, noch weiter bevorzugt von 0,8 bis 2,5 Gew.-%, am insbesondere von 1 bis 2 Gew.-%, bezogen auf das Gesamtgewicht des schweißhemmenden kosmetischen Mittels, verwendet wird.

Im Rahmen der vorliegenden Erfindung kann es auch vorgesehen sein, dass das schweißhemmende kosmetische Mittel als Öl-in-Wasser-Emulsion vorliegt. In diesem Fall wird das Mittel bevorzugt als treibmittelfreies Pumpspray oder Quetschspray versprüht oder als Roll-on appliziert. In diesem Zusammenhang ist es bevorzugt, wenn in dem in Form einer Öl-in-Wasser-Emulsion vorliegenden schweißhemmenden kosmetischen Mittel das Polysaccharid in einer Gesamtmenge von 0,05 bis 8 Gew.-%, vorzugsweise von 0,1 bis 7 Gew.-%, bevorzugt von 0,3 bis 5 Gew.-%, weiter bevorzugt von 0,5 bis 3 Gew.-%, noch weiter bevorzugt von 0,8 bis 2,5 Gew.-%, insbesondere von 1 bis 2 Gew.-%, bezogen auf das Gesamtgewicht des schweißhemmenden kosmetischen Mittels, verwendet wird.

Erfindungsgemäß kann es weiterhin vorgesehen sein, dass das schweißhemmende kosmetische Mittel als wässrige, wässrig-alkoholische oder wässrig-glykolische Lösung vorliegt.

Gemäß einer bevorzugten Ausführungsform der vorliegenden Erfindung wird in dem erfindungsgemäß kosmetischen Mittel Ethanol in einer Gesamtmenge von 1 bis 50 Gew.-%, vorzugsweise von 5 bis 40 Gew.-%, bevorzugt von 7 bis 35 Gew.-%, insbesondere von 10 bis 30 Gew.-%, bezogen auf das Gesamtgewicht des schweißhemmenden kosmetischen Mittels, eingesetzt. Es kann jedoch auch vorgesehen sein, dass in dem schweißhemmenden kosmetischen Mittel Ethanol in einer Gesamtmenge von 10 bis 95 Gew.-%, vorzugsweise von 15 bis 90 Gew.-%, bevorzugt von 20 bis 87 Gew.-%, weiter bevorzugt von 30 bis 85 Gew.-%, insbesondere von 40 bis 80 Gew.-%, bezogen auf das Gesamtgewicht des schweißhemmenden kosmetischen Mittels, eingesetzt wird.

Die Applikation des schweißhemmenden kosmetischen Mittels kann mittels verschiedener Verfahren erfolgen. Gemäß einer bevorzugten Ausführungsform ist das erfindungsgemäß kosmetische Mittel als Spray-Applikation konfektioniert. Die Spray-Applikation erfolgt mit einer Sprühvorrichtung, welche in einem Behälter eine Füllung aus dem flüssigen, viskos-fließfähigen, suspensionsförmigen oder pulverförmigen schweißhemmenden kosmetischen Mittel enthält. Die Füllung kann unter dem Druck eines Treibmittels stehen (Druckgasdosen, Druckgaspackungen, Aerosolpackungen), oder es kann sich um einen mechanisch zu bedienenden Pumpzerstäuber ohne Treibgas (Pumpsprays/ Quetschflasche) handeln. Die Behälter weisen eine Entnahmevorrichtung auf, bevorzugt in Gestalt von Ventilen, welche die Entnahme des Inhalts als Nebel, Rauch, Schaum, Pulver, Paste oder Flüssigkeitsstrahl ermöglichen. Als Behälter für die Sprühvorrichtungen kommen vor allem zylindrische Gefäße aus Metall (Aluminium, Weißblech, Rauminhalt bevorzugt maximal 1.000 ml), geschütztem bzw. nichtsplitterndem Glas oder Kunststoff (Rauminhalt bevorzugt maximal 220 ml) bzw. splitterndem Glas oder Kunststoff (Rauminhalt bevorzugt 50 bis 400 ml) in Frage. Cremeförmige, gelförmige, pastöse und flüssige Mittel können z.B. in Pump-, Spray- oder Quetschspendern abgepackt sein, insbesondere auch in Mehrkammer-Pump-, Mehrkammer-Spray- oder Mehrkammer-Quetschspendern. Die Verpackung für die Mittel kann undurchsichtig, aber auch transparent oder transluzent sein.

Das schweißhemmende kosmetische Mittel ist bevorzugt als Stift, Soft Solid, Creme, Roll-on, Dibenzylidenalditol-basiertes Gel, loses oder kompaktes Puder konfektioniert. Die Formulierung der schweißhemmenden kosmetischen Mittel in einer bestimmten Darreichungsform, wie beispielsweise einem Antitranspirant-Roll-on, einem Antitranspirantstift oder einem Antitranspirantgel, richtet sich bevorzugt nach den Anforderungen des Verwendungszwecks. Je nach Verwendungszweck können die schweißhemmenden kosmetischen Mittel daher in fester, halbfester, flüssiger, disperser, emulgierter, suspendierter, gelförmiger, mehrphasiger oder puderförmiger Form vorliegen. Unter den Begriff der Flüssigkeit fallen im Sinne der vorliegenden Erfindung auch jegliche Arten von Festkörperdispersionen in Flüssigkeiten. Weiterhin werden unter mehrphasigen schweißhemmenden kosmetischen Mitteln im Sinne der vorliegenden Erfindung Mittel verstanden, welche mindestens 2 verschiedene Phasen mit einer Phasentrennung aufweisen und bei welchen die Phasen horizontal, also übereinander, oder vertikal, also nebeneinander, angeordnet sein können.

Die Applikation kann beispielsweise mit einem Rollkugelapplikator erfolgen. Solche Roller weisen eine in einem Kugelbett gelagerte Kugel auf, welche durch Bewegung über eine Oberfläche bewegt werden kann. Dabei nimmt die Kugel etwas von dem zu verteilenden schweißhemmenden Mittel auf und befördert dieses an die zu behandelnde Oberfläche. Die Verpackung für die Mittel kann, wie zuvor ausgeführt, undurchsichtig, transparent oder transluzent sein.

Weiterhin ist es auch möglich, die schweißhemmenden kosmetischen Mittel mittels eines festen Stifts zu applizieren.

Die schweißhemmenden kosmetischen Mittel können darüber hinaus weitere Hilfsstoffe enthalten. Bevorzugt enthalten die schweißhemmenden kosmetischen Mittel mindestens einen weiten Hilfsstoff, ausgewählt aus der Gruppe von (i) Emulgatoren und/oder Tensiden; (ii) Hydrogelbildnern; (iii) Chelatbildnern; (iv) Deodorant-Wirkstoffen; (v) ein- und/oder mehrwertigen Alkoholen und/oder Polyethylenglycolen; (vi) hautkühlenden Wirkstoffen; (vii) Treibmitteln; (viii) anorganische lipophile Verdickungsmitteln sowie (ix) deren Mischungen.

Erfindungsgemäß bevorzugt geeignete Emulgatoren und Tenside sind ausgewählt aus anionischen, kationischen, nichtionischen, amphoteren, insbesondere ampholytischen und zwitterionischen Emulgatoren und Tensiden. Tenside sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8 bis 28 Kohlenstoffatomen, die gesättigt oder ungesättigt, linear oder verzweigt sein kann. Besonders bevorzugt ist diese C₈-C₂₈-Alkylkette linear.

Unter anionischen Tensiden werden Tenside mit ausschließlich anionischen Ladungen verstanden; sie enthalten z. B. Carboxylgruppen, Sulfonsäuregruppen oder Sulfatgruppen. Besonders bevorzugte anionische Tenside sind Alkylsulfate, Alkylethersulfate, Acylglutamate und C₈₋₂₄-Carbonsäuren sowie deren Salze, die sogenannten Seifen.

Unter kationischen Tensiden werden Tenside mit ausschließlich kationischen Ladungen verstanden; sie enthalten z. B. quartäre Ammoniumgruppen. Bevorzugt sind kationische Tenside vom Typ der quartären Ammoniumverbindungen, der Esterquats und der Amidoamine. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen.
Die amphoteren Tenside werden in ampholytische Tenside und zwitterionische Tenside unterteilt. Unter ampholytischen Tensiden werden solche oberflächenaktiven Verbindungen verstanden, die sowohl saure (beispielsweise -COOH oder -SO₃H-Gruppen) als auch basische hydrophile Gruppen (beispielsweise Aminogruppen) besitzen und sich also je nach Bedingung sauer oder basisch verhalten. Unter zwitterionischen Tensiden versteht der Fachmann Tenside, die im selben Molekül sowohl eine negative als auch eine positive Ladung tragen. Beispiele für bevorzugte zwitterionische Tenside sind die Betaine, die N-Alkyl-N,N-dimethylammonium-glycinate, die N-Acyl-aminopropyl-N,N-dimethylammoniumglycinate und die 2-Alkyl-3-carboxymethyl-3-hydroxyethyl-imidazoline mit jeweils 8 bis 24 Kohlenstoffatomen in der Alkylgruppe. Beispiele für bevorzugte ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropyl-glycine, N-Alkyltaurine, N-Alkylsarcosine, 2-Alkylaminopropionsäuren und Alkylaminoessigsäuren mit jeweils 8 bis 24 Kohlenstoffatomen in der Alkylgruppe

Die Zusammensetzungen, welche als Emulsion, insbesondere als Öl-in-Wasser-Emulsion, formuliert sind, enthalten bevorzugt mindestens einen nichtionischen Öl-in-Wasser-Emulgator mit einem HLB-Wert von mehr als 7 bis 20. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seite 913-916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist. In diesem Zusammenhang kann es erfindungsgemäß bevorzugt sein, wenn weiterhin ein Wasser-in-Öl-Emulgator mit einem HLB-Wert von größer 1,0 und kleiner/gleich 7,0 eingesetzt wird. Im Rahmen der vorliegenden Erfindung geeignete nichtionische Öl-in-Wasser-Emulgatoren und geeignete nichtionische Wasser-in-ÖI-Emulgatoren sind beispielsweise in der deutschen Offenlegungsschrift DE 10 2006 004 957 A1 beschrieben.

Weiterhin kann es von Vorteil sein, den schweißhemmenden kosmetischen Mitteln mindestens einen Chelatbildner, welcher ausgewählt ist aus Ethylendiamintetraessigsäure (EDTA) und ihren Salze sowie aus Nitrilotriessigsäure (NTA) und Mischungen dieser Substanzen, in einer Gesamtmenge von 0,01 bis 0,5 Gew.-%, vorzugsweise von 0,02 bis 0,3 Gew.-%, insbesondere von 0,05 bis 0,1 Gew.-%, bezogen auf das Gesamtgewicht des schweißhemmenden kosmetischen Mittels, zuzusetzen.

Die Antitranspirantwirkung der schweißhemmenden kosmetischen Mittel kann weitergehend gesteigert werden, wenn mindestens einen Deodorant-Wirkstoff in einer Gesamtmenge von 0,0001 bis 40 Gew.-%, vorzugsweise von 0,2 bis 20 Gew.-%, bevorzugt von 1 bis 15 Gew.-%, insbesondere von 1,5 bis 5 Gew.-%, bezogen auf das Gesamtgewicht des schweißhemmenden kosmetischen Mittels, eingesetzt wird. Sofern Ethanol in den kosmetischen Mitteln eingesetzt wird, gilt dieses im Rahmen der vorliegenden Erfindung nicht als Deodorant-Wirkstoff, sondern als Bestandteil des Trägers. Erfindungsgemäß bevorzugte Deodorant-Wirkstoffe sind ausgewählt aus der Gruppe der (i) Silbersalze; (ii) aromatischen Alkohole, insbesondere 2-Benzylheptan-1-ol sowie Mischungen von 2-Benzylheptan-1-ol und Phenoxyethanol; (iii) 1,2-Alkandiole mit 5 bis 12 Kohlenstoffatomen, insbesondere 3-(2-Ethylhexyloxy)-1,2-propandiol; (iv) Triethylcitrate; (v) Wirkstoffe gegen Exoesterasen, insbesondere gegen Arylsulfatase, Lipase, beta-Glucuronidase und Cystathion-β-lyase; (vi) kationischen Phospholipide; (vii) Geruchsabsorber, insbesondere Silicate, wie Montmorillonit, Kaolinit, Ilit, Beidellit, Nontronit, Saponit, Hectorit, Bentonit, Smectit und Talkum, Zeolithe, Zinkricinoleat, Cyclodextrine; (viii) desodorierend wirkenden Ionenaustauscher; (ix) keimhemmenden Mittel; (x) präbiotisch wirksamen Komponenten; sowie (xi) Mischungen dieser Deodorant-Wirkstoffe.

Bevorzugte schweißhemmenden kosmetische Mittel enthalten weiterhin mindestens ein wasserlösliches mehrwertiges C₂₋₉-Alkanol mit 2 bis 6 Hydroxylgruppen und/oder mindestens ein wasserlösliches Polyethylenglycol mit 3 bis 50 Ethylenoxid-Einheiten sowie Mischungen hiervon. Hierunter fallen nicht die vorstehend erwähnten Deodorant-Wirkstoffe in Form von 1,2-Alkandiolen. Bevorzugte Alkanole sind ausgewählt aus 1,2-Propylenglycol, 2-Methyl-1,3-propandiol, Glycerin, 1,2-Butylenglycol, 1,3-Butylenglycol, 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebige Isomeren-Gemische von cis- und trans-1,4-Dimethylolcyclohexan, sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind.

Gemäß einer weiteren Ausführungsform der vorliegenden Erfindung enthalten die schweißhemmenden kosmetischen Mittel weiterhin mindestens einen hautkühlenden Wirkstoff. Erfindungsgemäß geeignete hautkühlende Wirkstoffe sind beispielsweise Menthol, Isopulegol sowie Mentholderivate, z. B. Menthyllactat, Menthylglycolat, Menthyl Ethyl Oxamate, Menthylpyrrolidoncarbonsäure, Menthylmethylether, Menthoxypropandiol, Menthonglycerinacetal (9-Methyl-6-(1-methylethyl)-1,4-dioxaspiro (4.5)decan-2-methanol), Monomenthylsuccinat, 2-Hydroxymethyl-3,5,5-trimethylcyclohexanol und 5-Methyl-2-(1-methylethyl)cyclohexyl-N-ethyloxamat. Als hautkühlende Wirkstoffe bevorzugt sind Menthol, Isopulegol, Menthyllactat, Menthoxypropandiol, Menthylpyrrolidoncarbonsäure und 5-Methyl-2-(1-methylethyl)cyclohexyl-N-ethyloxamat sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.

Weiterhin kann es vorgesehen sein, dass die schweißhemmenden kosmetischen Mittel ein Treibmittel enthalten. In diesem Fall sind sie als treibgasgetriebenes Aerosol konfektioniert. Bevorzugte Treibmittel (Treibgase) sind Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, Tetrafluoropropene und zwar sowohl einzeln als auch in deren Mischungen. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann. Die Gesamtmenge der Treibmittel beträgt 20 bis 95 Gew.-%, vorzugsweise 30 bis 85 Gew.-%, insbesondere 40 bis 75 Gew.-%, jeweils bezogen auf das Gesamtgewicht des Antitranspirants, bestehend aus dem schweißhemmenden kosmetischen Mittel und dem Treibmittel. In diesem Zusammenhang kann es auch vorgesehen sein, dass die Gesamtmenge an Treibmittel 1 bis 95 Gew.-%, vorzugsweise 2 bis 85 Gew.-%, insbesondere 3 bis 75 Gew.-%, jeweils bezogen auf das Gesamtgewicht des Antitranspirants, bestehend aus dem schweißhemmenden kosmetischen Mittel und dem Treibmittel, beträgt.

Als Hilfstoffe können erfindungsgemäß weiterhin anorganische lipophile Verdickungsmittel eingesetzt werden. Bevorzugt ist das mindestens eine schweißhemmende Aluminiumsalz ungelöst in mindestens einem bei 20 °C und 1.013 hPa flüssigen kosmetischen Öl suspendiert. Zur besseren Anwendbarkeit kann dieser Suspension noch mindestens ein anorganisches lipophiles Verdickungsmittel als Suspendierhilfe zugesetzt werden. Erfindungsgemäß bevorzugte anorganische lipophile Verdickungsmittel sind ausgewählt aus hydrophobierten Tonmineralien und pyrogenen Kieselsäuren.

Die folgenden Beispiele erläutern die vorliegende Erfindung, ohne sie jedoch darauf einzuschränken:

### Beispiele:

### 1. Textilverfärbung und/oder Textilflecken

### 1.1 Testprodukte mit flüchtigen Ölen in der Ölphase

Als Testprodukt V-I wurde eine ölige Suspension, bestehend aus 33,3 Gew.-% aktiviertem Aluminiumchlorhydrat, 49,4 Gew.-% Cyclopentasiloxane, 6,67 Gew.-% Isopropylmyristat, 2,53 Gew.-% Bentone 38 V CG, 0,867 Gew.-% Propylenecarbonat, 6,67 Gew-% Parfum 233385 Bossanova und 0,500 Gew.-% 252479 Incap. Frag. No. 174917/ENC/200 hergestellt. Eine derartige Suspension ist unter anderem repräsentativ für wasserfreie Antitranspirant-Rollons, wasserfreie Antitranspirant-Wachsstifte und wasserfreie Antitranspirant-Sprays.

Zur Herstellung der erfindungsgemäßen Testprodukte werden 50 Gramm der obigen Mischung V-I mit jeweils 2 Gew.-% eines Polysaccharids gemischt. Die jeweils verwendeten Polysaccharide zur Herstellung der erfindungsgemäßen Testprodukte sind in nachfolgender Tabelle 1 wiedergegeben.

**Tabelle 1: erfindungsgemäße Testprodukte**

| Nr | Polysaccharid |
|---|---|
| E-I | Methylhydroxypropylcellulose (Methocel E4M) |
| E-II | Cetylhydroxyethylcellulose (Natrosol plus 330) |
| E-III* | Mikrokristalline Cellulose (Viavapur 105) |

| | |
|---|---|
| * nicht erfindungsgemäß | |

0,3 Gramm des jeweiligen Produkts V-I, E-I, E-II und E-III wurde direkt auf ein 10 x10 cm² großes Stück hellblauen Baumwollstoff (Polo Jersey, gewebt) aufgebracht, welches auf ein Waffelpiquehandtuch geheftet war. Nach einer Stunde Wartezeit wurde 1 ml einer künstlichen Schweißmischung (MgCl₂, CaCl₂, KCl, NaCl, Na₂SO₄, NaH₂PO₄, Glycin, Glucose, Milchsäure, Harnstoff; pH 5,2) aufgetragen und das Textil nach 24 Stunden Wartezeit (Alterung) in einem standardardisierten, haushaltsüblichen Waschprozess gewaschen (Miele W 1714) und maschinell getrocknet (Miele T 7644C). Die Waschbedingungen sind in untenstehender Tabelle 2 wiedergegeben.

**Tabelle 2: Parameter des Waschprozesses**

| | |
|---|---|
| Beladung: | 3,5 kg |
| Wassermenge: | 17 l |
| Temperatur: | 40 °C |
| Zeit Hauptwaschgang: | 1 h |
| Vorwäsche: | Ohne |
| Nachspülen: | 4 x |
| Waschmittel: | Spee Color Gel (Ch.: HH06.1.1UWM1.08:58) |
| Einwaage Waschmittel: | 75 ml (70 gr) |
| Weichspüler: | Ohne |
| Trocknerprogramm: | Extra Trocken - Baumwolle |

Die Produktauftragung und Waschung wurde insgesamt achtmal mit dem gleichen Textil wiederholt (entspricht 8 Anschmutz/Waschzyklen). Die Bewertung der Textilanschmutzung erfolgte visuell durch geschulte Laboranten anhand von Referenzbeispielen. Die Skala reichte von 0 (keine Flecken) bis 4 (sehr starke Fleckenbildung). Die Bewertung erfolgte direkt nach Abschluss der Waschreihe. Es wurden die in der Tabelle 3 angegebenen Ergebnisse erhalten:

**Tabelle 3: Ergebnisse der visuellen Rückstandsbewertung nach 8 Anschmutz/Waschzyklen**

| Produkt | Weiß | fettig |
|---|---|---|
| V-I | 1 | 1 |
| E-I | 0,7 | 0 |
| E-II | 0,5 | 0,5 |
| E-III* | 0,5 | 0,5 |

| | | |
|---|---|---|
| * nicht erfindungsgemäß | | |

Die erfindungsgemäßen Testrezepturen E-I und E-II, welche 2 Gew.-% eines speziellen Polysaccharids enthalten, zeigen gegenüber der Vergleichsrezeptur V-I ohne den Zusatz eines speziellen Polysaccharids eine deutlich reduzierte Bildung weißer und fettiger Flecken auf hellblauem Textil.

### 1.2 Testprodukte mit nichtflüchtigen Ölen in der Ölphase

Als Testprodukt V-II wurde eine ölige Suspension, bestehend aus 14,3 Gew.-% aktiviertem Aluminiumchlorhydrat, 68,0 Gew.-% 2-Ethylhexylpalmitat, 5,36 Gew.-% Triethylcitrat, 3,93 Gew.-% Bentone 38 V CG, 1,29 Gew.-% Propylenecarbonat und 7,14 Gew.-% Parfum TEU-E-1451 Padma w-on hergestellt. Eine derartige Suspension ist unter anderem repräsentativ für wasserfreie Antitranspirant-Roll-ons, wasserfreie Antitranspirant-Wachsstifte und wasserfreie Antitranspirant-Sprays.

Zur Herstellung der erfindungsgemäßen Testprodukte werden 50 Gramm der obigen Mischung V-II mit jeweils 2 Gew.-% eines Polysaccharids gemischt. Die jeweils verwendeten Polysaccharide zur Herstellung der erfindungsgemäßen Testprodukte sind in nachfolgender Tabelle 4 wiedergegeben.

**Tabelle 4: erfindungsgemäße Testprodukte**

| Nr | Polysaccharid |
|---|---|
| E-IV | Methylhydroxypropylcellulose (Methocel E4M) |
| E-V | Cetylhydroxyethylcellulose (Natrosol plus 330) |
| E-VI* | Mikrokristalline Cellulose (Viavapur 105) |

| | |
|---|---|
| * nicht erfindungsgemäß | |

0,3 Gramm des jeweiligen Produkts V-II, E-VI, E-V und E-VI wurde direkt auf ein 10 x10 cm² großes Stück hellblauen Baumwollstoff (Polo Jersey, gewebt) aufgebracht, welches auf ein Waffelpiquehandtuch geheftet war. Nach einer Stunde Wartezeit wurde 1 ml einer künstlichen Schweißmischung (MgCl₂, CaCl₂, KCl, NaCl, Na₂SO₄, NaH₂PO₄, Glycin, Glucose, Milchsäure, Harnstoff; pH 5,2) aufgetragen und das Textil nach 24 Stunden Wartezeit (Alterung) in einem standardardisierten, haushaltsüblichen Waschprozess gewaschen (Miele W 1714) und maschinell getrocknet (Miele T 7644C). Die Waschbedingungen sind in untenstehender Tabelle 5 wiedergegeben.

**Tabelle 5: Parameter des Waschprozesses**

| | |
|---|---|
| Beladung: | 3,5 kq |
| Wassermenge: | 17 l |
| Temperatur: | 40 °C |
| Zeit Hauptwaschgang: | 1 h |
| Vorwäsche: | Ohne |
| Nachspülen: | 4 x |
| Waschmittel: | Spee Color Gel (Ch.: HH06.1.1UWM1.08:58) |
| Einwaage Waschmittel: | 75 ml (70 gr) |
| Weichspüler: | Ohne |
| Trocknerprogramm: | Extra Trocken - Baumwolle |

Die Produktauftragung und Waschung wurde insgesamt achtmal mit dem gleichen Textil wiederholt (entspricht 8 Anschmutz/Waschzyklen). Die Bewertung der Textilanschmutzung erfolgte visuell durch geschulte Laboranten anhand von Referenzbeispielen. Die Skala reichte von 0 (keine Flecken) bis 4 (sehr starke Fleckenbildung). Die Bewertung erfolgte direkt nach Abschluss der Waschreihe. Es wurden die in der Tabelle 6 angegebenen Ergebnisse erhalten:

**Tabelle 6: Ergebnisse der visuellen Rückstandsbewertung nach 8 Anschmutz/Waschzyklen**

| Produkt | Weiß | fettig |
|---|---|---|
| V-II | 0 | 4 |
| E-IV | 0 | 3 |
| E-V | 0 | 3 |
| E-VI* | 0 | 3 |

| | | |
|---|---|---|
| * nicht erfindungsgemäß | | |

Die erfindungsgemäßen Testrezepturen E-VI und E-V, welche 2 Gew.-% eines speziellen Polysaccharids enthalten, zeigen gegenüber der Vergleichsrezeptur V-II ohne den Zusatz eines speziellen Polysaccharids eine deutlich reduzierte Bildung fettiger Flecken auf hellblauem Textil.

### 2. Formulierungsbeispiele

Die nachfolgenden Formulierungsbeispiele sollen den Gegenstand der Erfindung erläutern, ohne ihn hierauf zu beschränken. Das in den nachfolgenden Beispielen eingesetzte Polysaccharid ist ausgewählt aus der Gruppe von Hydroxyethylcellulose, Hydroxyethylmethylcellulose, Methylcellulose, Hydroxypropylmethylcellulose, Cetylhydroxyethylcellulose oder einer Mischung der vorgenannten Cellulosen.

### Schweißhemmende kosmetische Mittel (Mengenangaben in Gew.-%)

| | 1 | 2 | 3 | 4 | 5 | 6 |
|---|---|---|---|---|---|---|
| Hydrogenated Castor Oil | - | - | - | 1,5 | 1,5 | 1,5 |
| Stearyl Alcohol | 24,0 | 24,0 | 24,0 | 18 | 18 | 18 |
| Novata AB | - | - | - | 4 | 4 | 4 |
| Al-Zr-pentachlorohydrex Gly | 22,0 | 22,0 | 22,0 | 17,6 | 17,6 | 17,6 |
| Polysaccharid | 2,0 | 1,5 | 1,0 | 1,75 | 1,25 | 2,0 |
| PPG-14 Butyl Ether | 10,0 | 10,0 | 10,0 | 15,3 | 15,3 | 15,3 |
| gehärtetes Rizinusöl (z. B. Cutina HR) | 3,0 | 3,0 | 3,0 | - | - | - |
| Myristylmyristat | 1,5 | 1,5 | 1,5 | - | - | - |
| DL-Menthol | 0,2 | 0,2 | 0,2 | - | - | - |
| Eucalyptol | 0,2 | 0,2 | 0,2 | - | - | - |
| Anethol | 0,2 | 0,2 | 0,2 | - | - | - |
| Silica Dimethyl Silylate | 1,4 | 1,4 | 1,4 | - | - | - |
| Silica | 0,3 | 0,3 | 0,3 | - | - | - |
| Talkum | - | - | - | 3 | 3 | 3 |
| Emulgin B1 | - | - | - | 3 | 3 | 3 |
| Parfum | 2,0 | 2,0 | 2,0 | 1 | 1 | 1 |
| Cyclomethicone (mind. 95 Gew.-% Cyclopentasiloxan) | ad 100 | ad 100 | ad 100 | ad 100 | ad 100 | ad 100 |

### Schweißhemmende kosmetische Mittel in Form einer Öl-in-Wasser-Emulsion (Mengenangaben in Gew.-%)

| | 7 | 8 |
|---|---|---|
| Cutina ® AGS | 2,5 | 2,5 |
| Cutina® FS45 | 3,5 | 3,5 |
| Eumulgin® B₂ | 0,8 | 0,8 |
| Eumulgin® B3 | 0,8 | 0,8 |
| Diisopropyladipat | 6,0 | 6,0 |
| Novata® AB | 4,0 | 4,0 |
| Cutina® CP | 5,0 | 5,0 |
| Cutina® HR | 4,0 | 4,0 |
| Kesterwachs K62 | 5,0 | 5,0 |
| Locron® L (ACH-Lösung 50%ig) | 40 | 40 |

| Talkum Pharma G | 10 | 10 |
|---|---|---|
| Parfüm | 1,2 | 1,2 |
| 2-Benzylheptan-1-ol | - | 0,3 |
| Sensiva SC 50 | 0,6 | 0,6 |
| Polysaccharid | 2,0 | 3,0 |
| 1,2-Propandiol | 10 | 10 |
| Wasser, vollentsalzt | ad 100 | ad 100 |

### Schweißhemmende kosmetische Mittel in Form einer Mikroemulsionen (Angaben in Gew.-%)

| | 9 | 10 | 11 | 12 |
|---|---|---|---|---|
| Plantaren® 1200 | 1,7 | 1,7 | - | - |
| Plantaren® 2000 | 1,1 | 1,4 | 2,4 | 2,4 |
| Glycerinmonooleat | 0,71 | 0,71 | - | - |
| Dioctylether | 4,0 | 4,0 | 0,090 | 0,090 |
| Octyldodecanol | 1,0 | 1,0 | 0,020 | 0,020 |
| Parfümöl | 1,0 | 1,0 | 1,0 | 1,0 |
| Aluminiumchlorohydrat | 8,0 | 5,0 | 5,0 | 5,0 |
| 1,2-Propylenglycol | 5,0 | 5,0 | - | - |
| Glycerin | - | - | 5,0 | 5,0 |
| 2-Benzylheptan-1-ol | 0,50 | - | - | - |
| Triethylcitrat | - | 0,50 | 0,50 | 0,50 |
| Triclosan | 0,10 | - | - | - |
| Polysaccharid | 1,0 | 2,0 | 2,5 | 0,5 |
| Wasser | ad 100 | ad 100 | ad 100 | ad 100 |

### Schweißhemmende kosmetische Mittel in Form von Roll-ons (Mengenangaben in Gew.-%)

| | 13 | 14 | 15 | 16 |
|---|---|---|---|---|
| Ethanol 96 %-ig,(DEP vergällt) | 30 | 30 | 28 | 28 |
| Mergital® CS 11 | 2,0 | 2,0 | - | - |
| Eumulgin® B3 | 2,0 | 2,0 | 2,0 | 2,0 |
| Emulgin® B1 | - | - | 2,0 | 2,0 |
| Aluminiumchlorohydrat 50 % (Locron L) | 20 | 20 | 16 | 16 |
| Hydroxyethylcellulose | 0,50 | 0,50 | 0,30 | 0,30 |
| Polysaccharid | 2,5 | 0,50 | 2,0 | 1,5 |
| EDTA | - | - | - | 0,050 |
| Cocamidopropyl PG-Dimonium Chloride Phosphate | 0,20 | - | - | - |
| Parfümöl | 0,80 | 0,80 | 1,0 | 1,0 |
| Wasser | ad 100 | ad 100 | ad 100 | ad 100 |

### Schweißhemmende kosmetische Mittel in Form einer Wasser-in-Öl-Emulsion (Mengenangaben in Gew.-%)

| | 21 | 22 |
|---|---|---|
| Aluminiumchlorohydrat 50% in Wasser (Locron L) | 35,6 | 35,6 |
| 1,2-Propylenglycol | 13,0 | 13,0 |
| Cyclohexasiloxan | 6,00 | 6,00 |
| Finsolv TN | 8,00 | 8,00 |
| Abil EM 90 | 1,20 | 1,20 |
| Polyethylen-Wachs (MW= 500 g/mol, Smp = 83 bis 91 °C) | 10,0 | 10,0 |
| Polyalphaolefin-Wachs (MW = 1800 g/mol, Smp = 41 °C) | 0,100 | 0,100 |
| Polysaccharid | 2,00 | 0,500 |
| EDTA | - | 0,0500 |
| Wasser | 25,0 | 25,0 |
| Parfum | 1,00 | 1,00 |

### Schweißhemmende kosmetische Mittel (Mengenangaben in Gew.-%)

| | 23 | 24 |
|---|---|---|
| Cyclopentasiloxan | 14,0 | 14,0 |
| Abil EM 97 | 3,00 | 3,00 |
| Ethanol 96% | 10,0 | 10,0 |
| Aluminiumchlorohydrat 50% in Wasser (Locron L) | 40,0 | 40,0 |
| 1,2-Propylenglycol | 20,3 | 20,3 |
| Wasser | 11,6 | 11,6 |
| Polysaccharid | 2,00 | 0,500 |
| EDTA | - | 0,0750 |
| Parfum | 1,00 | 1,00 |

### Schweißhemmende kosmetische Mittel (Mengenangaben in Gew.-%, bezogen auf das Gesamtgewicht der treibmittelfreien Zusammensetzung)

| | 27 | 28 | 29 | 30 |
|---|---|---|---|---|
| Aluminiumchlorohydrate (ACH) | 28,6 | 14,29 | 32,11 | 28,57 |
| Bentone 38 V CG | 5,00 | 3,93 | 4,00 | 5,00 |
| Propylenecarbonate | 1,50 | 0,71 | 1,50 | 1,80 |
| Fragrance | 7,14 | 6,50 | 5,00 | 6,50 |
| 2-Ethylhexylpalmitate | - | 73,57 | - | - |
| Abil K 4 | 48,4 | - | - | - |
| Isopropylmyristate | 7,37 | - | 10,00 | 19,22 |
| Triethylcitrat | - | - | 10,5 | 19,2 |
| C10-C13 Isoalkane | - | - | 35,39 | 19,21 |
| Polysaccharid | 2,00 | 1,00 | 1,50 | 0,500 |

Die Beispielzusammensetzungen 27 bis 30 wurden in eine gegebenenfalls mit Epoxy-Phenollack beschichtete Aluminiumspraydose in einem Gewichtsverhältnis von Treibmittel (Butan/Propan/Isobutangemisch) zu Suspension von 80:20 bzw. 85:15 bzw. 60:40 bzw. 90:10 abgefüllt.

### Schweißhemmende kosmetische Mittel (Mengenangaben in Gew.-%, bezogen auf das Gesamtgewicht der treibmittelfreien Zusammensetzung)

| | 31 | 32 | 33 |
|---|---|---|---|
| Aluminiumchlorhydrat (ACH) | 33,0 | 33,0 | 33,0 |
| C₁₀-C₁₃-Isoalkan | 8,90 | 8,90 | 8,90 |
| Dow Corning ES-5227 DM | 1,40 | 1,40 | 1,40 |
| Isoceteth-20 | 0,500 | 0,500 | 0,500 |
| Dimethicone | 4,20 | 4,20 | 4,20 |
| Isopropylmyristat | 9,00 | 9,00 | 9,00 |
| 1,2 Propandiol | 7,00 | 25,0 | 25,0 |
| Phenoxyethanol | 0,500 | 0,500 | 0,500 |
| Parfüm | 2,50 | 2,50 | 2,50 |
| Polysaccharid | 2,00 | 0,500 | 1,50 |
| L-Menthol | 0,400 | 0,300 | - |
| trans-Anethol | - | 0,300 | - |
| Eucalyptol | - | 0,300 | - |
| Wasser | ad 100 | ad 100 | ad 100 |

Die Beispielzusammensetzungen 31 bis 33 wurden in eine gegebenenfalls mit Epoxy-Phenollack beschichtete Aluminiumspraydose in einem Gewichtsverhältnis von Treibmittel (Butan/Propan/Isobutangemisch) zu W/O-Emulsion von 80:20 bzw. 85:15 bzw. 60:40 bzw. 90:10 abgefüllt.

### Schweißhemmende kosmetische Mittel in Form von O/W-Emulsionen (Mengenangaben in Gew)

| | 34 | 35 | 36 |
|---|---|---|---|
| Aluminiumchlorhydrat (ACH) | 13,0 | 13,0 | 13,0 |
| Kalium Aluminiumsulfat KAl(SO₄)₂ · 12H₂O | 1,50 | 1,50 | 1,50 |
| Talk | 1,0 | - | - |
| Bentonite | - | 1,00 | - |
| Hectorite | - | - | 5,00 |
| Brij S 2 | 2,50 | 2,50 | 2,50 |
| Brij S 721 | 1,50 | 1,50 | 1,50 |
| Parfum | 1,10 | 1,10 | 1,10 |
| Arlamol E | 0,500 | 0,500 | 0,500 |
| Bisabolol | 0,100 | 0,100 | 0,100 |
| Dry Flo PC | 0,100 | 0,100 | 0,100 |
| Polysaccharid | 2,00 | 3,00 | 1,00 |
| Dow Corning 2501 Cosmetic Wax | 0,100 | 0,100 | 0,100 |
| Tocopherylacetat | 0,100 | 0,100 | 0,100 |
| Wasser | ad 100 | ad 100 | ad 100 |

### Schweißhemmende kosmetische Mittel (Mengenangaben in Gew.-%, bezogen auf das Gesamtgewicht der treibmittelfreien Zusammensetzung)

| | 37 | 38 | 39 | 40 |
|---|---|---|---|---|
| Aluminiumchlorhydrat (ACH) | 33,0 | 33,0 | 33,0 | 33,0 |
| Cyclomethicone | 12,0 | 9,40 | - | - |
| C10-C13-Isoalkan | - | - | 9,40 | 8,90 |
| Dow Corning ES-5227 DM | - | 1,40 | 1,40 | 1,40 |
| Abil EM 90 | 3,00 | - | - | - |
| Brij IC 20 | - | - | - | 0,500 |
| Dimethicone | - | 4,20 | 4,20 | 4,20 |
| Isopropylmyristat | 9,00 | 9,00 | 9,00 | 9,00 |
| Polysaccharid | 2,50 | 1,00 | 3,00 | 0,500 |
| 1,2 Propandiol | 7,00 | 7,00 | 7,00 | 7,00 |
| Phenoxyethanol | 0,500 | 0,500 | 0,500 | 0,500 |
| Parfüm | 2,50 | 2,50 | 2,50 | 2,50 |
| Wasser | add 100 | add 100 | add 100 | add 100 |

Die Beispielzusammensetzungen 37 bis 40 wurden in eine gegebenenfalls mit Epoxy-Phenollack beschichtete Aluminiumspraydose in einem Gewichtsverhältnis von Treibmittel (Butan/Propan/Isobutangemisch) zu W/O-Emulsion von 80:20 bzw. 85:15 bzw. 60:40 bzw. 90:10 abgefüllt.

### Schweißhemmende kosmetische Mittel in Form von W/O-Emulsionen (Mengenangaben in Gew.-%)

| | 41 | 42 | 43 | 44 |
|---|---|---|---|---|
| Aluminiumchlorohydrat 50 % (Locron L) | 62,5 | 62,5 | 60,0 | 58,0 |
| Propylenglycol | 5,00 | 5,00 | 7,50 | 9,50 |
| C12-C15 Alkylbenzoat | 8,04 | 8,04 | 8,04 | 8,04 |
| Dimethicone 2 cst | 6,43 | 6,43 | 6,43 | 6,43 |
| Dimethicone 5 cst | 1,57 | 1,57 | 1,57 | 1,57 |
| Polyethylen | 10,2 | 11,7 | 9,70 | 12,2 |
| Abil EM 90 | 0,998 | 0,998 | 0,998 | 0,998 |
| Abil EM 97 | 1,22 | 1,22 | 1,22 | 1,22 |
| Polysaccharid | 2,50 | 1,00 | 3,00 | 0,500 |
| Synthetisches Wachs | 0,100 | 0,100 | 0,100 | 0,100 |
| Parfüm | 1,50 | 1,50 | 1,20 | 1,50 |

Es wurden die folgenden Handelsprodukte eingesetzt:

| Handelsprodukt | INCI | Lieferant/Hersteller |
|---|---|---|
| Abil EM 90 | CETYL PEG/PPG-10/1 Dimethicone | Evonik |
| Abil EM 97 | Bis-PEG/PPG-14/14 Dimethicone, Cyclomethicone | Evonik |
| Abil K 4 | Cyclomethicone | Goldschmidt |
| Arlamol E | PPG-15 Stearyl ether | Croda |
| Bentone 38 V CG | Disteardimonium Hectorite | Elementis Specialities |
| Brij IC 20 | Isoceteth-20 | Croda |
| Brij S 2 | Steareth-2 | Croda |
| Brij S 721 | Steareth-21 | Croda |
| Cutina® CP | Cetyl Palmitate | BASF |
| Cutina® FS45 | Palmitic Acid, Stearic Acid | BASF |
| Cutina® HR | Hydrogenated Castor Oil | BASF |
| Dow Corning ® 245 | Cyclopentasiloxan | Dow Corning |
| Dow Corning ® 2501 | Bis-PEG-18 Methyl ether dimethyl silane | Dow Corning |
| Dow Corning ES-5227 DM | Dimethicone, PEG/PPG-18/18 Dimethicone im Gewichtsverhältnis 3:1 | Dow Corning |
| Dry Flo PC | Aluminum Starch Octenylsuccinate | National Starch |
| Eumulgin® B1 | Ceteareth-12 | BASF |
| Eumulgin® B2 | Ceteareth-20 | BASF |
| Eumulgin® B3 | Ceteareth-30 | BASF |
| Kesterwachs K62 | Cetearyl Behenate | Koster Keunen |
| Finsolv TN | C12-15 Alkyl Benzoate | Innospec |
| Locron L (AS = 50 %) | Aluminum Chlorohydrate | Clariant |
| Mergital® CS 11 | Ceteareth-11 | BASF |
| Novata® AB | Cocoglycerides (Schmelzpunkt 30 - 32 °C) | BASF |
| Plantaren® 1200 | LAURYL GLUCOSIDE, ca. 50 % AS | BASF |
| Plantaren® 2000 | DECYL GLUCOSIDE, ca. 50 % AS | BASF |
| Sensiva® SC 50 | 2-Ethylhexylglycerinether | Schülke & Mayr |

## Patentansprüche

1. Verwendung eines Polysaccharids, ausgewählt aus der Gruppe vnn C₁₋₁₀-alkylcellulosen, Hydroxy-C₂₋₁₀-alkylmethylcellulosen, Hydroxy-C₂₋₁₀-alkylethylcellulosen, Hydroxy-C₁₋₁₀-alkylcellulosen, Carboxy-C₁₋₁₀-alkylcelluosen und C₆₋₃₀-Alkylhydroxyethylcellulosen in schweißhemmenden kosmetischen Mitteln zur Reduzierung und/oder Verhinderung von während des Waschvorgangs auftretenden Textilverfärbungen und/oder Textilflecken.

## Claims

1. The use of a polysaccharide selected from the group of C₁₋₁₀ alkyl celluloses, hydroxy-C₂₋₁₀ alkyl methyl celluloses, hydroxy-C₂₋₁₀ alkyl ethyl celluloses, hydroxy-C₁₋₁₀ alkyl celluloses, carboxy-C₁₋₁₀ alkyl celluloses and C₆₋₃₀ alkyl hydroxyethyl celluloses in antiperspirant cosmetic agents for reducing and/or preventing textile discoloration and/or textile stains that occur during the washing process.

## Revendications

1. Utilisation d'un polysaccharide choisi dans le groupe des (C₁₋₁₀)alkylcelluloses, des hydroxy-(C₂₋₁₀)alkylméthylcelluloses, des hydroxy-(C₂₋₁₀)alkyléthylcelluloses, des hydroxy-(C₁₋₁₀)alkylcelluloses, des carboxy-(C₁₋₁₀)alkylcelluloses et des (C₆₋₃₀)alkylhydroxyéthylcelluloses dans des agents cosmétiques anti-transpirants pour réduire et/ou empêcher les décolorations de textiles et/ou les taches sur les textiles survenant pendant le processus de lavage.
